# EUROPEAN PATENT SPECIFICATION

(11) **EP 1 441 688 B1**
(45) Date of publication and mention of the grant of the patent: **28.05.2008**
(21) Application number: 02787566.5
(22) Date of filing: 05.11.2002
(51) Int. Cl.: A61Q 11/00, A61K 8/73, A61K 8/97, A61K 8/24, A61K 8/21

(54) **SOLID ORAL ANTI-TARTAR AND ANTI-PLAQUE COMPOSITIONS**
FESTE ORALE ANTI-ZAHNSTEIN- UND ANTI-PLAQUEZUBEREITUNGEN
COMPOSITIONS BUCCALES ANTITARTRE ET ANTIPLAQUE A L'ETAT SOLIDE

(30) Priority: 06.11.2001 IT MI20012320
(43) Date of publication of application: 04.08.2004
(73) Proprietor: Perfetti Van Melle S.p.A., 20020 Lainate (Milan) (IT)
(72) Inventor: COLLE, Roberto, I-20020 Lainate (MI) (IT); SALMOIRAGHI, Guglielmo, I-20020 Lainate (MI) (IT); BARRICA, Andrea, I-20020 Lainate (MI) (IT)
(74) Representative: Minoja, Fabrizio
(86) International application number: PCT/EP2002/012329
(87) International publication number: WO 2003/039503

(56) References cited:
- EP-A- 0 306 454
- WO-A-01/72274
- US-A- 4 512 968
- US-A- 4 627 977
- US-A- 4 753 792
- US-A- 5 281 410
- DATABASE CA [Online] CHEMICAL ABSTRACTS SERVICE, COLUMBUS, OHIO, US; NAKANAGA, HIROSHI ET AL: "Solid dentifrices" retrieved from STN Database accession no. 116:66968 XP002234594 & JP 03 255020 A (SANGI CO., LTD., JAPAN) 13 November 1991 (1991-11-13)

## Description

The present invention relates to oral anti-tartar and anti-plaque chewing gum compositions, useful as adjuvants in odontostomatological hygiene.

### BACKGROUND TO THE INVENTION

The problem of dental plaque and tartar formation has long been studied, and agents which can be used to combat and delay such formation are being actively researched.

The mechanisms that cause tartar deposits are well known; these deposits are constituted by calcium phosphate crystals which precipitate in the extracellular matrix of bacterial plaque. The pathogenetic role of tartar in periodontal diseases such as pyorrhoea, periodontitis, gingivitis and correlated disorders is equally well known.

Various substances have proved effective in reducing or preventing tartar formation and deposits on the teeth, including soluble pyrophosphates and polyphosphates, zinc salts, fluorides, diphosphonates, antibacterial agents such as triclosan, and abrasive agents such as silica or alumina. These substances, combined with one another in various ways, are included in the composition of most anti-tartar toothpastes now commercially available. The clinical efficacy of these toothpastes has been examined in numerous studies, reviewed in J. Clin. Dent. Vol IV(3), 71-81, 1993.

The most common toothpastes contain soluble polyphosphates associated with fluorides and silica, and possibly with polymers that possess bioadhesive properties, as described, for example, in US 4327977, US 4889713, US 5017362, US 5139769, US 4921693 and EP 492997.

Similar compositions, with the addition of antibacterial agents such as triclosan, are described, for example, in GB 2200551. In addition to toothpastes, chewing gums and candies with a similar composition have been developed. US 4 512 968 discloses a chewing gum comprising chitin and abrasives. US 4 627 977 discloses a chewing gum comprising NaF and polyphosphates.

The efficacy of these toothpastes, which has been the object of numerous studies (J. Clin. Dent. Vol. X(3), 99-102, 1999; Oral Surg. Oral Med. Oral Pathol., Vol. 70(4), 529-536, 1990; J. Clin. Dent. Vol. IX(4), 101-104, 1998), is due to inhibition of calcium phosphate precipitation by the polyphosphates that complex the calcium ions in the saliva, to the abrasive action of silica, to the reinforcing effect of fluorides on the tooth enamel and to the action of bioadhesive polymers, where used, which protects the mucosae and causes slow release of the other ingredients.

The polymers most often used in compositions designed to control tartar are polycarboxylates derived from acrylic or methacrylic acid, particularly copolymers of maleic anhydride with methyl vinyl ether (GANTREX ®). However, these polymers are not approved for use in foodstuffs, which means that they can only be used to make toothpastes and mouthwashes.

On the other hand, chewing gum designed as adjuvants in dental hygiene and oral hygiene in general, which have properties that can be described as anti-tartar, anti-decay, whitening and/or refreshing, are becoming increasingly popular. The main advantage of these forms of administration is that they can be used freely and conveniently during the day in any place and on any occasion, in addition to that fact that the release of the active elements (functional ingredients) is slower and more regular than in the case of an ordinary toothpaste.

### DESCRIPTION OF THE INVENTION

The present invention relates to chewing gum oral formulations in a solid form, whose efficacy is superior to that of similar known formulations.

The compositions of the invention contain effective amounts of:
a. polyphosphates, preferably a mixture of alkali metal pyrophosphates and triphosphates;
b. an abrasive agent (preferably hydrated silica);
c. a source of fluoride ions;
d. a polymer derived from chitin, or other naturally occurring hydrocolloids or a mixture thereof;
e. vegetable extracts;
f. optionally antibacterial or disinfectant agents.

In addition to the active ingredients referred to above, the compositions of the invention will contain excipients suitable to define the final form of administration.

Thus, for example, a chewing gum formulation will require a suitable base consisting of gum base, sweeteners, polyalcohols such as xylitol, sorbitol and mannitol, flavourings, dyes, softeners, plasticisers, stabilisers, thickeners, etc.

The fact that the compositions of the invention comprise a system able to form a film on the oral mucosa increases protection against tartar deposits, because the active ingredients remain in contact with the user's teeth and gums for a longer time and the polyphosphates are protected against the hydrolysing action of the oral cavity.

In the present invention, this system consists of a chitin deacetylated derivative, possibly chemically modified and optionally in association with other polymers, to enhance its bioadhesive properties and its ability to protect the polyphosphates against hydrolysing agents.

There has been great scientific interest in controlled-release systems directed at the oral mucosa in the past decade (J. Clin. Phar. Ther. (2000) 25, 21-42). The polymers studied include partly deacetylated chitin, highly deacetylated chitin or chitosan and hydrolysed chitosan or oligosaccharide, which have proved able to adhere to the tissues thanks to the positive charges of the ammonium groups. Partly as a result of their bioadhesive properties, these polymers accelerate wound healing and haemostasis (Biom. 1999, 20(22): 2139-45; J. Oral. Max. Surg. 57: 49-52). Specific studies demonstrate the bioadhesive properties of chitosan towards the oral mucosa (Biom. 16 (1995) 617-624; J. Control Rel. 61: 175-183, Int. J. Pharm. 73: 43-48).

Among the forms described, the preferred form is chitosan oligosaccharide, a commercially available compound that comprises two to seven monomer D-glucosamine units bonded to one another with β -1,4 bonds, mainly obtained by enzymatic hydrolysis of chitosan with a higher molecular weight.

Although this procedure is known, it does not appear to have been applied in compositions similar to those which are the object of this invention.

Optionally, naturally occurring polysaccharides hydrocolloids may be used as alternative with similar bioadhesive properties. Polysaccharides hydrocolloids of this type are: xanthan gum, locust bean gum, alginates, carrageneen, gallan gum and others.

Broadly, the polymer-based system described above amounts to between 0.5 and 5% by weight on the total composition, and preferably between 1 and 3%.

The polyphosphates used in the compositions may be alkali metal pyrophosphates (diphosphates), hexametaphosphates, tripolyphosphates or mixtures thereof. A mixture of disodium diacid diphosphate and pentasodium or pentapotassium triphosphate is particularly preferred. It has been proved that a toothpaste containing this mixture provides a more marked reduction of tartar than a toothpaste containing pyrophosphates not associated with triphosphates (J. Clin. Dent. Vol. IX(4), 101-104, 1998).

Broadly, the polyphosphates amount to between 0.5 and 5% by weight on the total composition to which the invention relates.

The function of the abrasive agent is to increase the plaque-removing action already possessed by ordinary chewing gum. It may be formed by hydrated silica (in a suitable form), calcium carbonate (in a suitable form) or talc, either individually or combined with one another. These abrasives may also be present totally or partly, either individually or in a mixture thereof, in encapsulated form, in particular encapsulated in calcium alginate. Chewing gum containing microgranules of hydrated silica encapsulated in calcium alginate has proved more effective in removing plaque than a chewing gum with the same formulation but without microgranules (Doc. Os 06.2001 779-781). According to the present invention, the encapsulated microgranules may contain dyes, flavourings, functional ingredients and herb extracts. This abrasive agent is usually present in percentages of between 0.5 and 7% by weight.

Suitable sources of fluoride ions include sodium fluoride, potassium fluoride, ammonium fluoride, sodium monofluoro-phosphate and other known non-toxic salts containing fluorine, in concentrations which provide fluoride percentages of between 0.005 and 0.2% by weight.

The vegetable extracts present in the compositions of the invention are selected from extracts of *Centella asiatics, Malva sylvestris, Melaleuca alternifolia, Commiphora abyssinica* (myrrh), *Krameria triandra (*rhatany*), Acacia catechu, Medicago sativa* (alfalfa), resins of the genus *Styrax,* such as *Styrax benzoin* (benzoin), *Matricaria recutita* (camomile), *Echinacea purpurea* (echinacea) and *Croton lechleri* (dragon's blood). Extracts of these plants, whose activity has been known for some time, are commercially available.

The combination with these extracts gives the formulations anti-inflammatory/decongestant, emollient, wound-healing, antiseptic and astringent properties. These properties are desirable in at least two respects in the ambit of the present invention:
firstly, to assist and reinforce the reduction in diseases of the oral mucosa caused by the reduction in tartar, and
secondly, to control and prevent contact stomatitis similar to that manifested with the use of toothpastes, known as "toothpaste stomatitis", in particularly predisposed persons.

In the formulations of the invention, these extracts may be encapsulated in alginate together with the abrasive agent.

Said extracts may be added to the formulations in percentages of between 0.01 and 2% by weight.

The formulations of the invention can be prepared by conventional techniques, by adding and mixing the various ingredients to the gum base in the case of chewing gum, which may then undergo coating operations in accordance with equally conventional techniques.

The formulations of the invention may include disinfectant or antibacterial agents such as triclosan, zinc salts or zinc oxide, either alone or combined with one another, in concentrations of between 0.1 and 5% by weight. These agents are designed to combat the formation of bacterial plaque, which leads to tartar deposits.

The formulations of the invention may also include decorative crystals, preferably consisting of gum arabic and dyes deposited on the surface of the product with a purely aesthetic function.

Daily use of the chewing gum in accordance with the invention reduces tartar deposits and has other beneficial effects on the condition of the oral and gingival mucosa.

The following examples illustrate the invention in greater detail.

### EXAMPLE 1

### Coated chewing gum weighing 1.4 g.

| Percentage composition (by weight) | |
|---|---|
| Ingredient | % |
| Gum base | 24.5 |
| Xylitol | 23.5 |
| Sorbitol | 23.2 |
| Mannitol | 16 |
| Flavouring | 1.8 |
| Silicon dioxide | 3 |
| Gum arabic | 1 |
| Glycerin | 1 |
| Disodium diacid diphosphate | 1 |
| Pentasodium triphosphate | 1 |
| Chitosan oligosaccharide | 1 |
| Maltitol syrup | 0.93 |
| Titanium dioxide (E171) | 0.7 |
| Quick Coat | 0.6 |
| Aspartame | 0.6 |
| Decorative crystals | 0.05 |
| Acesulfame | 0.05 |
| Carnauba wax | 0.05 |
| Potassium fluoride | 0.02 |
| | 100 |

### EXAMPLE 2

### Coated chewing gum weighing 1.4 g with vegetable extracts.

| Percentage composition (by weight) | |
|---|---|
| Ingredient | % |
| Gum base | 24.5 |
| Xylitol | 23.5 |
| Sorbitol | 23.2 |
| Mannitol | 16 |
| Flavouring (*) | 1.8 |
| Silicon dioxide | 3 |
| Gum arabic | 1 |
| Glycerin | 1 |
| Disodium diacid diphosphate | 1 |
| Pentasodium triphosphate | 1 |
| Chitosan oligosaccharide | 1 |
| Maltitol syrup | 0.93 |
| Titanium dioxide (E171) | 0.7 |
| Quick Coat | 0.6 |
| Aspartame | 0.6 |
| Acesulfame | 0.05 |
| Carnauba wax | 0.05 |
| Potassium fluoride | 0.02 |
| Mallow, myrrh, centella, melaleuca, rhatany and acacia catechu extracts. | 0.05 |
| | |
| Total | 100 |

### EXAMPLE 3

### Efficacy tests: reduction in tartar deposit.

A double-blind crossover clinical trial has been conducted to compare the effects of a chewing gum in accordance with Example 1 with those of a placebo gum.

28 Adults were admitted to the trial and treated with two chewing gums for five minutes, four times a day, for 6 weeks. At the end of this period a quantitative evaluation of the tartar deposit was carried out in accordance with the modified Volpe and Manhold index (J. Periodont. Res. (Suppl.) 14:31-60, 1974). Throughout the treatment period, the patients all used the same toothpaste (not containing anti-tartar agents) and followed a similar diet. The same patients were then treated for six weeks immediately after the first evaluation of the tartar deposit with the other chewing gum (Example 1 or placebo) in accordance with the same treatment procedure as before. At the end of the treatment period a second quantitative evaluation of the tartar deposit was carried out in accordance with the same procedure as described above.

The results were subjected to statistical analysis using Student's two-tailed paired sample "t" test.

The evaluation conducted after the patients had chewed the gum described in Example 1 demonstrated a 13.9% reduction in tartar deposits compared with those observed after chewing of the placebo gum. This reduction is statistically significant. The results of the study are summarised in Table 1.

**Table 1**

| *"T" test: paired samples for means* | | |
|---|---|---|
| | *Placebo* | *Ex. 1* |
| Mean | 4.2410714 | 3.6517857 |
| Variance | 10.539269 | 7.9599041 |
| Observations | 28 | 28 |
| Pearson's correlation | | 0.9858011 |
| Hypothesised difference of means | | 0 |
| P(T<=t) two-tailed | | 6.884-05 |

## Claims

1. A chewing-gum formulation comprising:
a. a mixture of alkali metal pyrophosphates and tripolyphosphates;
b. an abrasive agent;
c. a source of fluoride ions;
d. a partly or totally deacetylated derivative of chitin, optionally in association with a naturally-occurring hydrocolloid;
e. vegetable extracts selected from *Centella asiatica, Malva sylvestris, Melaleuca alternifolia, Commiphora abyssinica (*myrrh*)*, *Krameria triandra (*rhatany*)*, *Acacia catechu, Medicago sativa* (alfalfa), resins of the genus *Styrax,* such as *Styrax benzoin* (benzoin), *Matricaria recutita* (camomile), *Echinacea purpurea* (echinacea) and *Croton lechleri* (dragon's blood);
f. optionally antibacterial/disinfectant agents.

2. Chewing-gum formulations as claimed in claim 1, further containing excipients selected from gum base, sweeteners, polyalcohols, flavourings, dyes, softeners, plasticisers, stabilisers and thickeners.

3. Chewing-gum formulations as claimed in claim 1 or 2, wherein the abrasive agent is hydrated silica, calcium carbonate and talc, either individually or in a mixture thereof.

4. Chewing-gum formulations as claimed in claim 1, wherein the chitin-derivative is a chitosan.

5. Chewing-gum formulations as claimed in claim 4, wherein the chitosan is chitosan oligosaccharide.

6. Chewing-gum formulations as claimed in any one of claims 1 to 5, wherein the naturally occurring hydrocolloids are xanthan gum, locust bean gum, alginates, carrageeneens, gellan gum or other polysaccharides with bioadhesive properties.

7. Chewing-gum formulations as claimed in any one of claims 1 to 6, containing 0.5 to 5% by weight of alkali metal pyrophosphates and tripolyphosphates mixture, 0.5 to 7% by weight of an abrasive agent (possibly wholly or partly encapsulated), 0.5 to 5% by weight of partly or totally deacetylated derivative of chitin, and a source of fluoride ions able to guarantee a fluoride intake of 0.005 to 0.2% .

8. Chewing-gum formulations as claimed in claim 7, containing 0.01 to 2% by weight of vegetable extracts.

9. Chewing-gum formulations as claimed in any one of claims 1 to 8, comprising disinfectant and/or antibacterial agents selected from triclosan, zinc oxide and zinc salts, either alone or in combination with one another, in concentrations of between 0.1 % and 5%.

## Patentansprüche

1. Eine Kaugummi-Zubereitung umfassend:
a. ein Gemisch aus Alkalimetall-Pyrophosphaten und -Tripolyphosphaten;
b. ein Schleifmittel;
c. eine Quelle für Fluoridionen;
d. ein teilweise oder vollständig deacetyliertes Derivat von Chitin, gegebenenfalls in Verbindung mit einem natürlich vorkommenden Hydrokolloid;
e. Pflanzenextrakte, ausgewählt aus *Centella asiatica, Malva sylvestris, Melaleuca alternifolia, Commiphora abyssinica* (Myrrhe), *Krameria triandra* (Ratanhia), *Acacia catechu, Medicago sativa* (Alfalfa), Harze der Art *Styrax,* wie *Styrax benzoin* (Benzoe), *Matricaria recutita* (Kamille), *Echinacea purpurea* (Echinacea), und *Croton lechleri* (Drachenblut);
f. gegebenenfalls antibakterielle Mittel/Desinfektionsmittel.

2. Kaugummi-Zubereitungen nach Anspruch 1, zusätzlich Hilfsstoffe enthaltend, ausgewählt aus Gummibasis, Süßstoffen, Polyalkoholen, Aromastoffen, Färbemitteln, Weichmachern, Plastifizierungsmitteln, Stabilisatoren und Verdickungsmitteln.

3. Kaugummi-Zubereitungen nach Anspruch 1 oder 2, wobei das Schleifmittel ein hydriertes Silica, Calciumcarbonat und Talk, entweder einzeln oder in einem Gemisch davon, ist.

4. Kaugummi-Zubereitungen nach Anspruch 1, wobei das Chitinderivat ein Chitosan ist.

5. Kaugummi-Zubereitungen nach Anspruch 4, wobei das Chitosan ein Chitosanoligosaccharid ist.

6. Kaugummi-Zubereitungen nach einem der Ansprüche 1 bis 5, wobei die natürlich vorkommenden Hydrokolloide Xanthangummi, Johannesbrotgummi, Alginate, Carrageene, Gellangummi oder andere Polysaccharide mit bioadhesiven Eigenschaften sind.

7. Kaugummi-Zubereitungen nach einem der Ansprüche 1 bis 6, enthaltend 0,5 bis 5 Gew.-% eines Gemischs aus Alkalimetall-Pyrophosphaten und -Tripolyphosphaten, 0,5 bis 7 Gew.-% eines Schleifmittels (gegebenenfalls vollständig oder teilweise verkapselt), 0,5 bis 5 Gew.-% eines teilweise oder vollständig deacetylierten Derivates von Chitin und eine Quelle von Fluoridionen, die eine Fluoridaufnahme von 0,005 bis 0,2 % sicherstellt.

8. Kaugummi-Zubereitungen nach Anspruch 7, enthaltend 0,01 bis 2 Gew.-% Pflanzenextrakte.

9. Kaugummi-Zubereitungen nach einem der Ansprüche 1 bis 8, umfassend Desinfektionsmittel und/oder antibakterielle Mittel, ausgewählt aus Triclosan, Zinkoxid und Zinksalzen, entweder allein oder in Kombination miteinander, in Konzentrationen zwischen 0,1 % bis 5 %.

## Revendications

1. Formulation de gomme à mâcher comprenant :
a. un mélange de pyrophosphates et de tripolyphosphates de métaux alcalins ;
b. un agent abrasif ;
c. une source d'ions fluorure ;
d. un dérivé de chitine partiellement ou totalement désacétylé, facultativement en association avec un hydrocolloïde naturel ;
e. des extraits végétaux choisis parmi *Centella asiatica, Malva sylvestris, Melaleuca alternifolia, Commiphora abyssinica* (myrrhe), *Krameria triandra* (ratanhia), *Acacia catechu, Medicago sativa* (luzerne), des résines du genre *Styrax,* telles que *Styrax benzoin* (benzoïne), *Matricaria recutita* (camomille), *Echinacea purpurea* (échinacée) et *Croton lechieri* (sang de dragon) ;
f. facultativement des agents antibactériens/désinfectants.

2. Formulations de gomme à mâcher selon la revendication 1, contenant en outre des excipients choisis parmi la base de gomme, des édulcorants, des poly(alcools), des arômes, des teintes, des ramollissants, des plastifiants, des stabilisants et des épaississants.

3. Formulations de gomme à mâcher selon la revendication 1 ou 2, dans lesquelles l'agent abrasif est de la silice hydratée, du carbonate de calcium et du talc, soit individuellement, soit dans un mélange de ceux-ci.

4. Formulations de gomme à mâcher selon la revendication 1, dans lesquelles le dérivé de chitine est un chitosan.

5. Formulations de gomme à mâcher selon la revendication 4, dans lesquelles le chitosan est un oligosaccharide de chitosan.

6. Formulations de gomme à mâcher selon l'une quelconque des revendications 1 à 5, dans lesquelles les hydrocolloïdes naturels sont de la gomme de xanthane, de la gomme de caroube, des alginates, des carragénines, de la gomme gellan ou d'autres polysaccharides avec des propriétés bioadhésives.

7. Formulations de gomme à mâcher selon l'une quelconque des revendications 1 à 6, contenant 0,5 à 5 % en poids de mélange de pyrophosphates et de tripolyphosphates de métaux alcalins, 0,5 à 7 % en poids d'un agent abrasif (éventuellement totalement ou partiellement encapsulé), 0,5 à 5 % en poids de dérivé de chitine partiellement ou totalement désacétylé, et une source d'ions fluorure capable de garantir une admission de fluorure de 0,005 à 0,2 %.

8. Formulations de gomme à mâcher selon la revendication 7, contenant 0,01 à 2 % en poids d'extraits végétaux.

9. Formulations de gomme à mâcher selon l'une quelconque des revendications 1 à 8, comprenant des agents désinfectants et/ou antibactériens choisis parmi le triclosan, l'oxyde de zinc et des sels de zinc, soit seuls, soit en combinaison les uns avec les autres, en des concentrations comprises entre 0,1 % et 5 %.
